# EUROPEAN PATENT APPLICATION

(11) **EP 2 898 815 A1**
(43) Date of publication of application: **29.07.2015**
(21) Application number: 13817235.8
(22) Date of filing: 12.07.2013
(51) Int. Cl.: A61B 1/00, A61M 25/01, G02B 23/24

(54) **GUIDE TUBE, GUIDE DEVICE, AND METHOD FOR USING GUIDE DEVICE**

(30) Priority: 13.07.2012 JP 2012157558
(71) Applicant: Kimura, Masashi, Nishinomiya-shi, Hyogo 669-1103 (JP)
(72) Inventor: Kimura, Masashi, Nishinomiya-shi, Hyogo 669-1103 (JP)
(74) Representative: Viering, Jentschura & Partner Patent- und Rechtsanwälte
(86) International application number: PCT/JP2013/069127
(87) International publication number: WO 2014/010722

(57) **Abstract**

A guide tube 10 according to the present invention has an initial form designed in advance according to a shape of an internal space 30. When an external force is applied, the guide tube deforms from the initial form to another form. When the applied external force is removed, the guide tube returns to the initial form. The guide tube 10 includes a first end portion 11. The first end portion 11 is preferably closed.

## Description

### [TECHNICAL FIELD]

The present invention relates to guide tubes, guide devices, and methods for using a guide device.

### [BACKGROUND ART]

Endoscopes have been used as means for observing a portion of interest present in an internal space of a subject. For example, medical fields use an endoscope to observe or treat an organ in a humans' body cavity. Alternatively, industrial fields use an endoscope to check the inside of equipment or piping.

Observation through the endoscope is carried out in a manner that a thin and long insertion portion having an imaging function is inserted into an internal space of a subject. However, where the internal space winds, it is difficult to change the forward direction of the insertion portion in conformity with the winding direction by simply inserting and pushing the insertion portion of the endoscope by a hand. The forward movement of the insertion portion may accordingly be inhibited at the winding point. As a result, it is difficult to insert the insertion portion up to a target point where the portion of interest can be observed. In view of the foregoing, an advancing direction changing device was proposed that makes the tip end of the insertion portion of the endoscope movable (see Patent Literature 1).

The advancing direction changing device disclosed in Patent Literature 1 includes an angle knob and a traction wire. The angle knob is mounted on an endoscope handling section and is connected to a pulley. The traction wire has one end connected to the tip end of an endoscope insertion portion and the other end wound to the pulley. When the angle knob of the endoscope handling section is operated to pull the traction wire, the traction wire is operated to change the direction of the tip end of the endoscope insertion portion to any desired direction. This can facilitate insertion of the endoscope insertion portion even when the internal space winds.

### [CITATION LIST]

### [Patent Literature]

[Patent Literature 1] Japanese Patent Application Laid-Open Publication No. 7-136104.

### [SUMMARY OF INVENTION]

### [Technical Problem]

However, the endoscope including the advancing direction changing device in Patent Literature 1 is complicated in structure and high in purchase and maintenance costs, resulting in hesitation in employment. Besides, the torque of the pulley may drill a human's body cavity or damage fibers.

The present invention has been made in view of the foregoing problems, and has its object of providing a guide tube that forms a to-be-delivered object delivery path through which a to-be-delivered object is delivered for a predetermined operation on a portion of interest in an internal space.

### [Solution to Problem]

A guide tube according to the present invention has an initial form designed in advance according to an internal space. When an external force is applied to the guide tube, the guide tube deforms from the initial form to another form. When the external force is removed, the guide tube returns to the initial form.

In an embodiment, the guide tube includes at least one curved portion.

In one embodiment, the guide tube includes a straight portion, a first curved portion, and a second curved portion. The straight portion continues to the first curved portion, and the first curved portion continues to the second curved portion. The first curved portion extends away from an axial line of the straight portion from an end of the straight portion. The second curved portion extends to approach the axial line of the straight portion from an end of the first curved portion.

In an embodiment, the guide tube includes a first end portion. The first end portion is closed.

In an embodiment, a closing member configured to close the first end portion is provided at the first end portion.

In an embodiment, the closing member is detachable.

In an embodiment, the guide tube includes an inner guide tube and an outer guide tube.

In an embodiment, an imaging device is provided at one or each of the inner and outer guide tubes.

A guide device according to the present invention includes any one of the above guide tube and a form changing means configured to apply an external force to the guide tube.

In an embodiment, the guide tube includes an inner guide tube and an outer guide tube. The inner guide tube is inserted in the outer guide tube.

In an embodiment, the inner guide tube includes a first cuff, and the outer guide tube includes a second cuff.

In an embodiment, the guide device further includes an imaging device provided at one or each of the inner and outer guide tubes.

In one embodiment, the form changing means is a stem.

In one embodiment, the form changing means is a fluid.

A method for using a guide device according to the present invention is a method for using any one of the above guide devices including: deforming the guide tube from the initial form to the other form and maintaining the guide tube in the other form by applying an external force to the guide tube using the form changing means; inserting the guide tube into the internal space; returning the guide tube to the initial form by removing the external force applied to the guide tube; and adjusting a position of the guide tube.

In one embodiment, the returning guide tube to the initial form and the adjusting a position of the guide tube are performed in plural stages.

### [Advantageous Effects of Invention]

The guide tube according to the present invention forms a to-be-delivered object delivery path through which a to-be-delivered object is delivered for a predetermined operation on a portion of interest in an internal space. When the delivery path for the insertion portion of the endoscope is formed using the guide tube according to the present invention, the insertion portion can be inserted with ease into the internal space of a subject even if the tip end of the insertion portion of the endoscope is immovable.

### [BRIEF DESCRIPTION OF DRAWINGS]

[FIG. 1] FIGS. 1A and 1B are illustrations explaining an embodiment of a guide tube according to the present invention.
[FIG. 2] FIGS. 2A and 2B are illustrations explaining an internal space.
[FIG. 3] FIGS. 3A and 3B are illustrations explaining another embodiment of the guide tube according to the present invention.
[FIG. 4] FIGS. 4A-4D are illustrations explaining another embodiment of the guide tube according to the present invention.
[FIG. 5] FIG. 5 is a schematic illustration explaining still another embodiment of the guide tube according to the present invention.
[FIG. 6] Fig. 6 is a schematic illustration showing an embodiment of a guide device according to the present invention.
[FIG. 7] FIGS. 7A-7D are illustrations explaining a method for using the guide device in the embodiment.
[FIG. 8] FIG. 8 is a schematic illustration showing the first embodiment of the guide device according to the present invention.
[FIG. 9A] FIGS. 9A and 9B are illustrations explaining a method for using the guide device according to the embodiment.
[FIG. 9B] FIGS. 9C and 9D are illustrations explaining the method for using the guide device according to the embodiment.
[FIG. 10] FIG. 10 is a schematic illustration showing the second embodiment of the guide device according to the present invention.
[FIG. 11] FIG. 11 is a schematic illustration showing the guide device employed in observation of an oviduct as a predetermined operation.

### [DESCRIPTION OF EMBODIMENTS]

With reference to the accompanying drawings, a description will be given below about a guide tube, a guide device, and a method for using a guide device according to the present invention. It should be noted that the present invention is not limited to the following embodiments.

### [Basic principle]

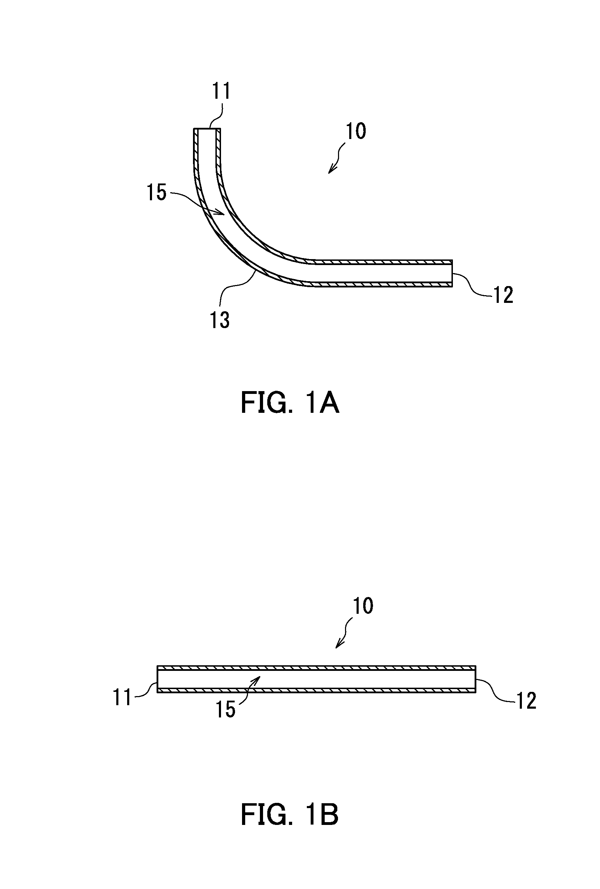

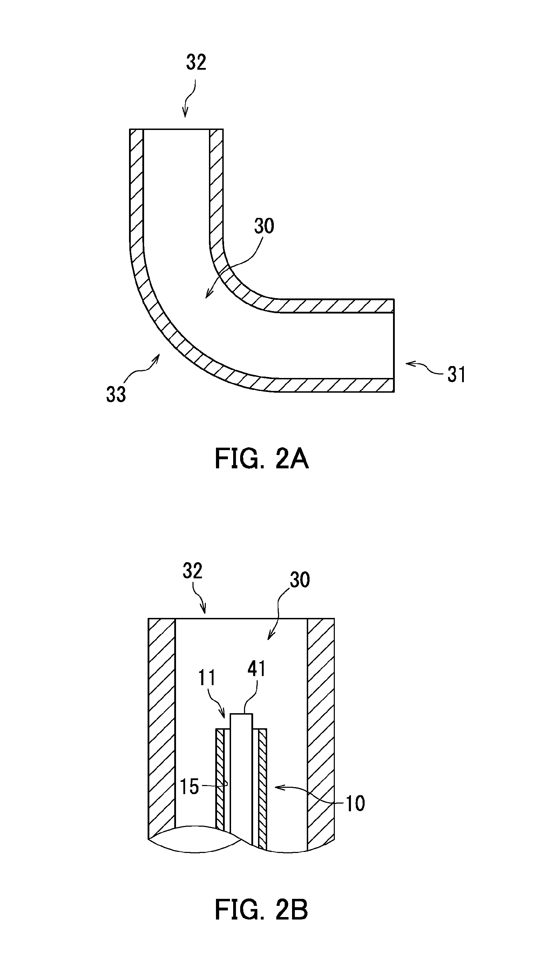

FIGS. 1A and 1B are illustrations explaining an embodiment of a guide tube 10 according to the present invention. FIG. 1A is a schematic illustration showing a cross section of a guide tube 10 in an initial form. FIG. 1B is a schematic illustration showing a cross section of the guide tube 10 in another form. FIGS. 2A and 2B are illustrations explaining an internal space 30. FIG. 2A is a schematic illustration showing a cross section of the internal space 30. FIG. 2B is a schematic illustration showing a to-be-delivered object delivered to the internal space 30 through the guide tube 10.

The guide tube 10 is used to form a delivery path in a manner to be inserted into the internal space 30. The delivery path is a path through which a to-be-delivered object is carried to a target point within the internal space 30 or carried out from the internal space 30. The to-be-delivered object is delivered up to the target point within the internal space 30 through the delivery path, and then performs a predetermined operation (e.g., observation or treatment) on a portion of interest within the internal space 30.

The guide tube 10 and the internal space 30 will be descried in detail with reference to FIGS. 1 and 2. The guide tube 10 has an initial form. When an external force is applied to the guide tube 10, the guide tube 10 deforms from the initial form to another form. Further, when the external force applied to the guide tube 10 is removed, the guide tube 10 returns to the initial form. The initial form of the guide tube 10 is designed in advance according to the shape of the internal space 30. Specifically, the initial form of the guide tube 10 is designed so as to conform with a winding shape of the internal space 30.

FIG. 2A shows an internal space of a pipe as an example of the internal space 30. The internal space 30 has one end portion 31, the other end portion 32, and a winding portion 33. In the present embodiment, an example configuration of the guide tube 10 will be discussed that is applicable to a case where the other end portion 32 is a portion of interest.

The guide tube 10 includes a first end portion 11 and a second end portion 12. The guide tube 10 has a curved portion 13 curved in the initial form such that when the guide tube 10 is inserted into the internal space 30, the first end portion 11 of the guide tube 10 passes on the winding portion 33 of the internal space 30 and moves forward to the other end portion 32. A tube inner space 15 is surrounded by the curved portion 13 in the guide tube 10.

As such, the guide tube 10 in the present embodiment has the initial form designed in advance according to the shape of the internal space 30. Further, the characteristic of the guide tube 10 is such that the guide tube 10 deforms when an external force is received and returns to the initial form when the external force is removed. Accordingly, by controlling the external force applied to the guide tube 10 to deform the guide tube 10 in conformity with the shape of the internal space 30, the guide tube 10 can be inserted so that the first end portion 11 can be favorably inserted up to a target point in the internal space 30.

The tube inner space 15 of the guide tube 10 inserted in the internal space 30 forms a delivery path through which a to-be-delivered object is delivered. Thus, the to-be-delivered object can be inserted from the second end portion 12 of the guide tube 10 and delivered to the internal space 30 through the tube inner space 15. For example, the to-be-delivered object may be an insertion portion 41 of an endoscope. As shown in FIG. 2B, when the guide tube 10 in the present embodiment is used for observation of the other end portion 32 (at the portion of interest) using the insertion portion 41 of the endoscope, the insertion portion 41 of the endoscope can be inserted up to a location (target point) where the other end portion 32 can be observed. Further, the tube inner space 15 of the guide tube 10 forms the smooth delivery path. This can prevent inhibition of the forward movement of the insertion portion 41 around a winding part of the internal space 30. As a result, even in the case using an endoscope having an insertion portion 41 of which tip end is immovable, the insertion portion 41 can be inserted smoothly into the internal space 30.

The guide tube 10 may be made of silicone, polyethylene, or vinyl chloride, for example. Among them, vinyl chloride is preferable from the view point of cost. The guide tube 10 may be transparent or opaque.

The outer diameter of the guide tube 10 can be appropriately set according to an internal space 30 to which the guide tube is applied, and may be 1.0 mm to 10.0 mm, for example. Where the internal space 30 is a nasal cavity, the outer diameter of the guide tube 10 is preferably 2.0 mm to 6.0 mm, and especially preferably 3.8 mm. Note that the outer diameter of the guide tube 10 may not necessarily be constant. For example, the outer diameter of the guide tube 10 may continuously or irregularly vary.

The inner diameter of the guide tube 10 (diameter of the tube inner space 15) can be set appropriately according to a to-be-delivered object that is to be delivered, and may be 0.9 mm to 5.0 mm, for example. For example, in the case where the to-be-delivered object is a nasal endoscope for observation of a throat or a larynx, the inner diameter of the guide tube 10 is preferably 1.0 mm to 3.0 mm, and especially preferably 2.0 mm. Note that the inner diameter of the guide tube 10 may not necessarily be constant. For example, the inner diameter of the guide tube 10 may continuously or irregularly vary.

The length of the guide tube 10 may be appropriately set according to an internal space 30 to which the guide tube 10 is applied and a to-be-delivered object that is to be delivered. Specifically, in order to form an delivery path for a nasal endoscope used for observation of a throat or a larynx, the length of the guide tube 10 is preferably 200.0 mm to 500.0 mm, and especially preferably 350.0 mm.

The guide tube 10 according to the present embodiment has been described with reference to FIGS. 1 and 2. In the present embodiment, the internal space of the pipe and the insertion portion of the endoscope have been discussed as examples of the internal space 30 and the to-be-delivered object, respectively. However, as noted above, the present invention is not limited to them.

The internal space 30 may be a body cavity of a living body of a human, an animal, etc. in typical cases. Alternatively, it may be a space inside a machine, a structure, etc. The to-be-delivered object may be selected according to an operation in the internal space 30. For example, in observation of the internal space 30, the to-be-delivered object may be an imaging device such as an endoscope. Alternatively, in delivery to a body cavity of a living body, the to-be-delivered object may be a medicine, air (e.g., oxygen), or a tag (maker). Any to-be-delivered object is applicable to the present invention as long as it can be delivered, sucked, inhaled, circulated, or ventilated through the guide tube 10.

### [First Embodiment of Guide Tube]

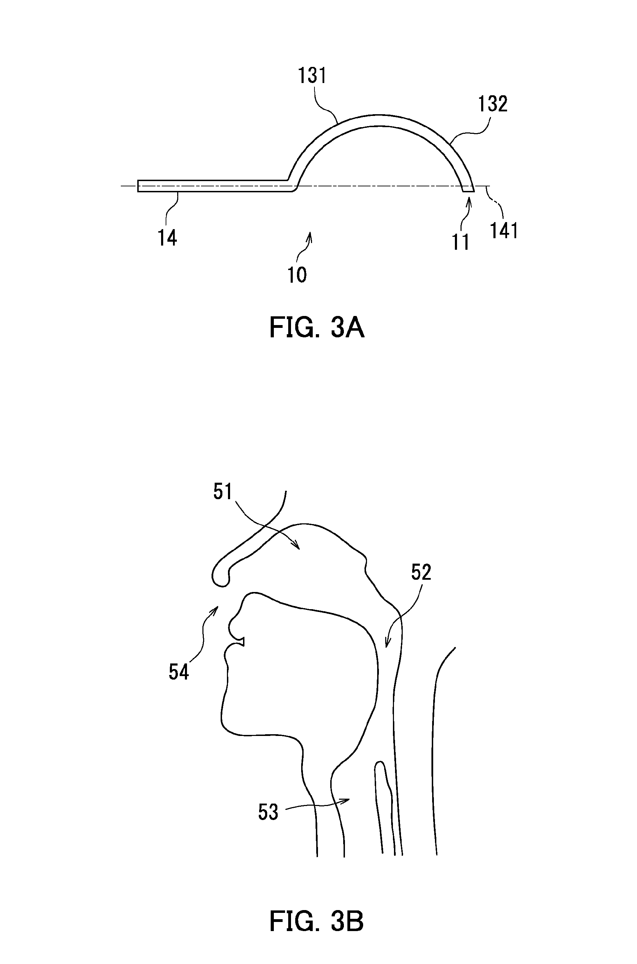

FIGS. 3A and 3B are illustrations explaining another embodiment of the guide tube 10 according to the present invention. FIG. 3A is a schematic illustration showing the initial form of the guide tube 10. FIG. 3B is a schematic illustration showing a nasal cavity 51, a pharyngeal cavity 52, and a laryngeal cavity 53. With reference to FIGS. 3A and 3B, the guide tube 10 will be described below. Note that the guide tube 10 in the present embodiment has the same configuration as the guide tube 10 in the aforementioned embodiment except that the initial form is different, and therefore, duplicate description will be omitted.

The nasal cavity 51 corresponds to the internal space 30 in the present embodiment. In order to observe a patient's state of deglutition, a nasal endoscope is used that is to pass through the nasal cavity 51. The tip end of the insertion portion of the nasal endoscope is inserted from the nostril 54 and allowed to pass through the nasal cavity 51 to reach the vicinity of the inlet of the pharyngeal cavity 52 (target point) for observation of the pharyngeal cavity 52 or the laryngeal cavity 53 (i.e., a portion of interest).

The guide tube 10 in the present embodiment has the initial form designed in advance according to the shape of the nasal cavity 51 in order to form a path for delivery of the nasal endoscope. Specifically, the guide tube 10 includes in the initial form a straight portion 14, a first curved portion 131, and a second curved portion 132. The straight portion 14 continues to the first curved portion 131. The first curved portion 131 continues to the second curved portion 132. The first curved portion 131 extends away from an axial line 141 of the straight portion 14 from the end of the straight portion 14. The second curved portion 132 extends to approach the axial line 141 of the straight portion 14 from the end of the first curved portion 131.

The guide tube 10 has the initial form in conformity with the shape from the nasal cavity 51 to the inlet of the pharyngeal cavity 52. Further, the characteristic of the guide tube 10 is such that the guide tube 10 deforms when an external force is received and returns to the initial form when the external force is removed. Accordingly, when the external force applied to the guide tube 10 is controlled to deform the guide tube 10 so as to conform with the shape of the nasal cavity 51 in the process of inserting the guide tube 10 up to the inlet of the pharyngeal cavity 52 through the nasal cavity 51, the guide tube 10 can be inserted favorably through the nasal cavity 51. Specifically, the guide tube 10 can be inserted into the nasal cavity 51 from the nostril 54, while directing the first end portion 11 of the guide tube 10 obliquely upward, and then moved forward to the pharyngeal cavity 52, while directing the first end portion 11 of the guide tube 10 downward. As a result, the tube inner space 15 of the guide tube 10 forms the path for delivery of the nasal endoscope. Through the tube inner space 15, the tip end of the insertion portion of the nasal endoscope can be inserted up to the vicinity of the pharyngeal cavity 52. Note that the nasal endoscope has been discussed as a to-be-delivered object herein, which however, should not be taken to limit the present invention. The to-be-delivered object may be any other object, such as air, medicine, a syringe.

The first and second end portions 11 and 12 of the guide tube 10 are open in the above embodiment. Accordingly, the to-be-delivered object delivered from the second end portion can be released from the first end portion 11. As a result, when the to-be-delivered object comes in contact with a portion of interest in the internal space, a predetermined operation can be carried out on the portion of interest. For example, administration of a medicine to a portion of interest in a body cavity can enable treatment on the portion of interest.

### [Second Embodiment of Guide Tube]

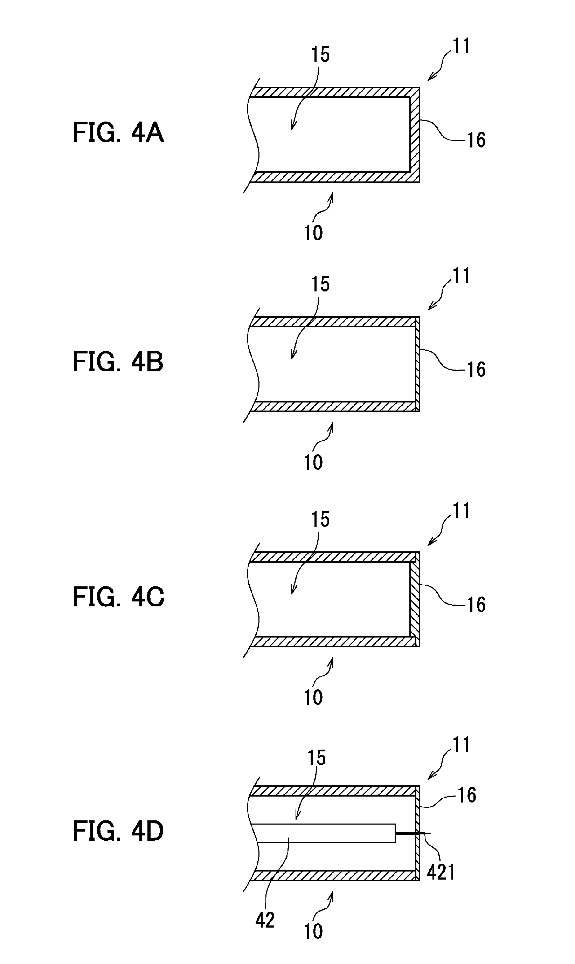

FIGS. 4A-4D are illustrations explaining another embodiment of the guide tube 10 according to the present invention. FIGS. 4A-4C are schematic illustrations showing a closed structure of the guide tube 10. FIG. 4D is a schematic illustration showing the guide tube 10 when in use. In the present embodiment, the first end portion 11 of the guide tube 10 is closed. The guide tube 10 according to the present embodiment will be described below with reference to FIGS. 4A-4D. The guide tube 10 has a similar configuration as that in the embodiment described with reference to FIGS. 1-3 except that the first end portion 11 is closed. Therefore, duplicate description will be omitted.

Specifically, the first end portion 11 of the guide tube 10 is closed by a closing member 16 provided at the first end portion 11. For example, as shown in FIG. 4A, the closing member 16 is integrally formed with the first end portion 11 of the guide tube 10 so as to close the first end portion 11. Alternatively, the closing member 16 may be provided as an independent component. For example, as shown in FIG 4B, the closing member 16 may be made of rubber, plastic, or a metal film. Alternatively, as shown in FIG. 4C, the closing member 16 is a cap capable of being attached to the first end portion 11. The closing member 16 formed as an independent component is attached to the first end portion 11 to close the first end portion 11. The closing member 16 is attached to the first end portion 11 by means of adhesion, fitting, or screwing, for example. The closing member 16 preferably seals the first end portion 11. In addition, it is preferable that the closing member 16 is detachably attached to the first end portion 11.

For example, when a body cavity is observed through an endoscope using the guide tube 10 in the present embodiment, the endoscope can be out of contact with the body cavity in the observation. Being out of contact with the body cavity, the endoscope can kept in a sterilized state. This can eliminate the need of disinfection and sterilization for preventing nosocomial infection. Thus, expensive facilities for disinfection and sterilization can be dispensed with. When the guide tube 10 is applied to observation through an endoscope, an optical lens can be employed as the closing member.

As shown in FIG. 4D, where the closing member 16 provided at the first end portion 11 of the guide tube 10 is a film, a syringe 42 is employable as a to-be-delivered object to be delivered. The syringe 42 can administrate a medicine to a body cavity in a manner to be delivered to the body cavity through the guide tube 10 and pierce the film with a needle 421. When the syringe 42 is enclosed within the guide tube 10 by the film, contamination of the syringe 42 in administration can be reduced to a minimum, thereby reducing a time-consuming job for disinfection of the syringe 42.

The guide tube 10 of the present embodiment has been described with reference to FIGS. 4A-4D. In the present embodiment, the first end portion 11 of the guide tube 10 is closed, so that the to-be-delivered object can be delivered to the internal space with the to-be-delivered object isolated from the internal space. Thus, contamination of the to-be-delivered object can be reduced.

### [Third Embodiment of Guide Tube]

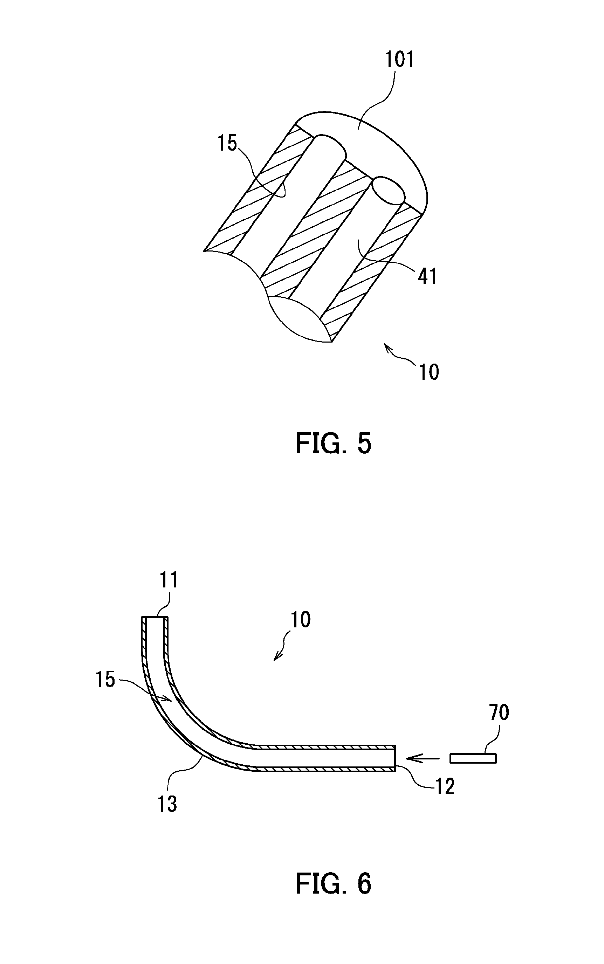

FIG. 5 is a schematic illustration explaining still another embodiment of the guide tube 10 according to the present invention. In the present embodiment, the guide tube 10 includes an insertion portion 41 of an endoscope. The guide tube 10 according to the present embodiment will be described below with reference to FIG. 5.

The guide tube 10 includes a main body 101. The main body 101 may be made of silicone, polyethylene, or vinyl chloride, as described above. A tube inner space 15 is formed in the longitudinal direction of the main body 101. The insertion portion 41 of the endoscope is integrally embedded in the main body 101.

The guide tube 10 of the present embodiment has been described with reference to FIG. 5. In the present embodiment, the guide tube 10 includes the insertion portion 41 of the endoscope. Accordingly, only required in observation through the insertion portion 41 of the endoscope is to insert the guide tube 10 into the internal space, thereby eliminating the need of inserting the insertion portion 41 into the tube inner space 15.

### [Basic Principle of Guide Device]

FIG. 6 is a schematic illustration showing a guide device 20 according to the present embodiment. The guide device 20 includes a guide tube 10 and a form changing means 70. The guide tube in the embodiment described with reference to FIGS. 1 and 2 is employed as the guide tube in the present embodiment. With reference to FIG. 6, the guide device 20 will be described below.

The form changing means 70 applies an external force to the guide tube 10. The form changing means 70 is a solid or a fluid such as a liquid or air. When being sent into the tube inner space 15, the form changing means 70 applies the external force to the guide tube 10, thereby deforming the guide tube 10.

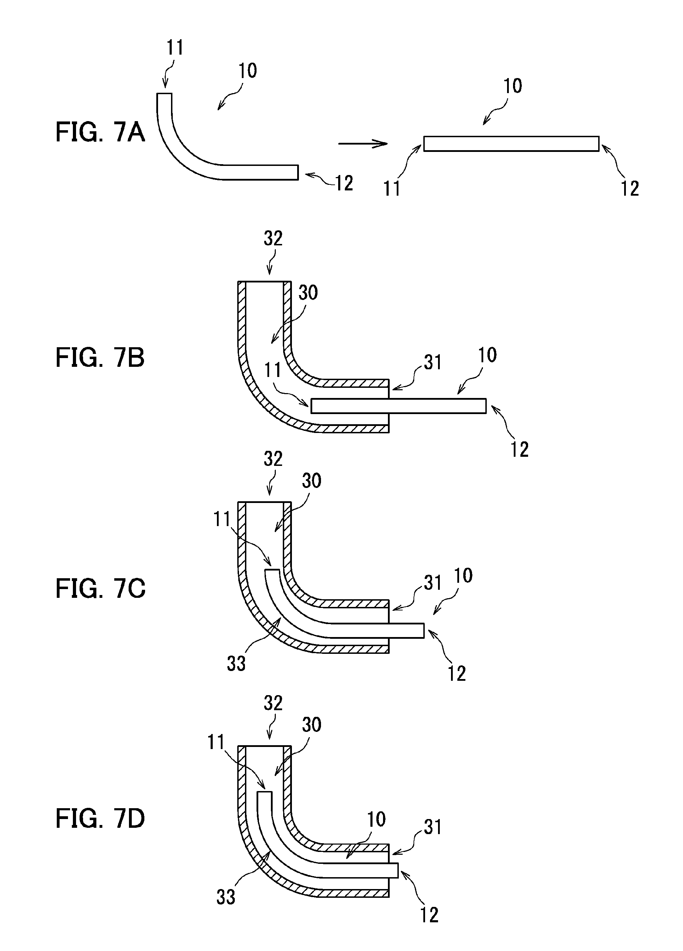

FIGS. 7A-7D are illustrations explaining a method for using the guide device 20 according to the present embodiment. FIGS. 7A-7D are schematic illustrations showing respective steps of inserting the guide tube 10 of the guide device 20 into the internal space 30. With reference to FIGS. 6 and 7, the method for using the guide device 20 according to the present embodiment will be described below. The guide device 20 is operated through the steps S 11-S 14.

Step S11: As shown in FIG. 7A, the form changing means 70 applies the external force to the guide tube 10 to deform the guide tube 10 from the initial form to another form. Further, the form changing means 70 maintains the guide tube 10 in the other form. In the present embodiment, the curved portion 13 of the guide tube 10 is deformed to be straight and maintained so as to facilitate insertion of the guide tube 10 into the internal space 30.

Step S12: As shown in FIG. 7B, the first end portion 11 of the guide tube 10 is inserted into the internal space 30. Specifically, the first end portion 11 is inserted up to the winding portion 33.

Step S13: As shown in FIG. 7C, the external force applied to the guide tube 10 is removed to return the guide tube 10 from the other form to the initial form. In the present embodiment, when the applied external force is removed, the curved portion 13 of the guide tube 10 returns from the straight form to a curved form in conformity with the shape of the winding portion 33 of the internal space 30.

Step S14: As shown in FIG. 7D, the position of the guide tube 10 in the returned curved form is adjusted so that the first end portion 11 reaches the target point in the internal space 30. Specifically, the position adjustment is performed in a manner that the guide tube 10 is further inserted, pulled out, or rotated so as to conform with the shape of the internal space 30.

The method for using the guide device 20 has been described with reference to FIGS. 6 and 7. According to this use method, when the guide tube 10 of the guide device 20 is inserted in the internal space 30, the path for delivery of the to-be-delivered object can be formed. Thus, the to-be-delivered object can be inserted from the second end portion 12 of the guide tube 10 and delivered to the internal space 30 through the tube inner space 15.

Note that in Steps S13 and S14, the external force applied to the guide tube 10 is removed at once to directly return the guide tube 10 to the initial form, and then, the position of the guide tube 10 is adjusted so that the first end portion 11 reaches the target point in the internal space 30, which however, should not be taken to limit the present embodiment. Steps S13 and S14 may be performed in plural stages until it the guide tube 10 returns to the initial form from the other form, in a manner that the external force is removed step by step to deform the guide tube 10 to intermediate forms, and the position of the guide tube 10 in the intermediate forms is adjusted at each stage.

Noted that the external force can be removed step by step by controlling the strength of the external force applied to the guide tube 10, or by controlling a part of the guide tube 10 where the external force is applied, for example. When the position of the guide tube 10 is adjusted by deforming the guide tube 10 step by step to the intermediate forms for conformity with the winding shape of the internal space 30, the guide tube 10 can be inserted smoothly into the internal space 30 even when the internal space 30 is narrow or complicatedly winds. Where the form changing means 70 is a fluid, the first end portion 11 of the guide tube 10 is preferably closed.

### [First Embodiment of Guide Device]

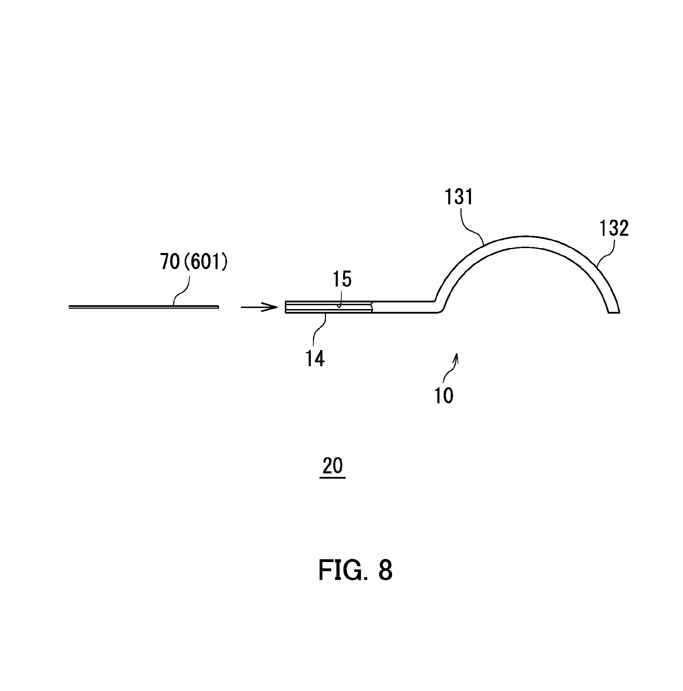

FIG. 8 is a schematic illustration showing the first embodiment of the guide device 20 according to the present invention. The nasal cavity 51 corresponds to the internal space 30 in the present embodiment. With reference to FIG. 8, the guide device 20 will be described below. The guide device 20 includes the guide tube 10 and the form changing means 70.

The guide tube 10 forms a path for delivery of a nasal endoscope used for observation of the pharyngeal cavity 52 or laryngeal cavity 53. The guide tube 10 has an initial form similar to that of the guide tube 10 in the embodiment described with reference to FIGS. 3A and 3B.

The form changing means 70 is a straight stem 701. When the stem 701 is inserted in the tube inner space 15 of the guide tube 10 to apply the external force to the guide tube 10, the guide tube 10 can be deformed to a straight form. The stem 701 is made of plastic.

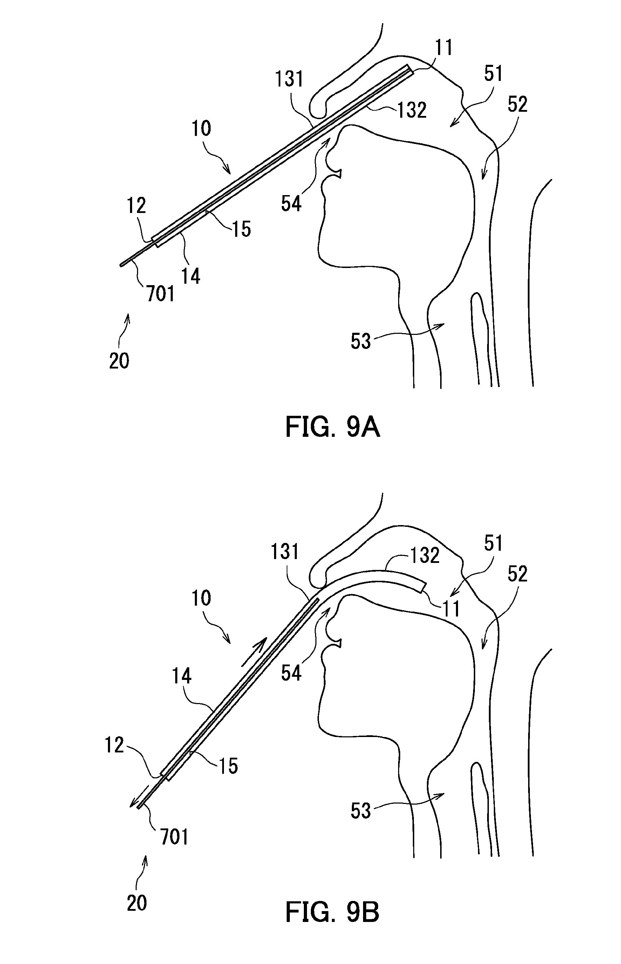

FIGS. 9A-9D are illustrations explaining a method for using the guide device 20 according to the present embodiment. FIG. 9A-9D are schematic illustrations showing operation steps of the guide device 20. The method for using the guide device 20 will be descried below with reference to FIGS. 8, 9A, and 9B. The guide device 20 is operated through Steps S21-S26.

Step S21: As shown in FIG. 9A, the stem 701 is inserted into the tube inner space 15 of the guide tube 10 so as to deform the guide tube 10 from the initial form to a straight form. Further, the guide tube 10 is maintained in the straight form. Specifically, the stem 701 deforms first and second curved portions 131 and 132 of the guide tube 10 straight, and maintains them in the straight shape.

Step S22: The first end portion 11 of the guide tube 10 deformed in the straight form is inserted obliquely upward into the nasal cavity 51 from the nostril 54. Specifically, a part of the guide tube 10 up to the second curved portion 132 is inserted in the nasal cavity 51. The stem 701 is inserted in the nasal cavity 51 along with the second curved portion 132.

Step S23: As shown in FIG. 9B, an end portion of the stem 701 inserted in the nasal cavity 51 along with the second curved portion 132 is pulled out from the guide tube 10. When the part of the stem 701 is pulled out, the external force applied to the second curved portion 132 is removed. Accordingly, the second curved portion 132 returns from the straight form to the initial form to be curved downward. As a result, the first end portion 11 faces the nasal cavity 51.

Step 24: The second curved portion 132 is further pushed in to move the first end portion 11 forward from the nasal cavity 51 to the pharyngeal cavity 52 or the laryngeal cavity 53. Having been returned to the curved form in conformity with the shape of the nasal cavity 51 in Step S23, the second curved portion 132 can be smoothly inserted into the nasal cavity 51 in Step 24.

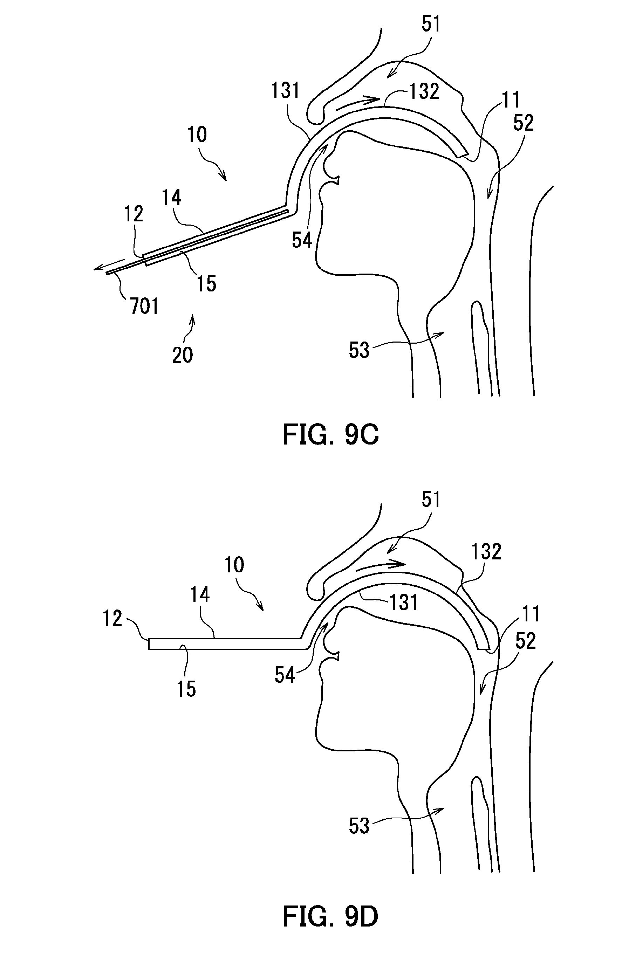

Step S25: As shown in FIG. 9C, the stem 701 is further pulled out from the guide tube 10 to return the first curved portion 131 to the initial form from the straight form.

Step S26: As shown in FIG. 9D, the first curved portion 131 is pushed up to the nasal cavity 51 so that the first end portion 11 reaches the vicinity of the inlet of the pharyngeal cavity 52. Having been returned to the curved form in conformity with the shape of the nasal cavity 51 in Step S25, the first curved portion 131 can be smoothly inserted into the nasal cavity 51 in Step 26.

The method for using the guide device 20 has been descried above with reference to FIGS. 8 and 9A-9D. According to the use method, insertion of the guide tube 10 of the guide device 20 in the nasal cavity 51 can form the path for delivery of the insertion portion of the nasal endoscope. When the nasal endoscope is inserted from the second end portion 12 of the guide tube 10 and delivered to the nasal cavity 51 through the tube inner space 15, the insertion portion of the nasal endoscope can observe the larynx behind the back of the pharyngeal cavity 52.

Note that Steps S23 and S24 may be performed in plural stages in a manner that the second curved portion 132 is inserted into the nasal cavity 51 step by step. Also note that Steps S25 and S26 may be performed in plural stages in a manner that the first curved portion 131 is inserted into the nasal cavity 51 step by step.

Furthermore, the material of the stem 701 can be appropriately selected according to the strength required for deforming the guide tube 10 from the initial form to another form. For example, the stem 701 may be made of metal, plastic, or rubber. The stem 701 may be made of an elastic macromolecular material. The form of the stem 701 may be a form other than the straight form (e.g., a curved form or an irregular form) according to the shape of any other predetermined space. In addition, the stem 701 can function as a tongue depressor for depressing the root of a tongue, thereby further facilitating insertion of the guide tube 10.

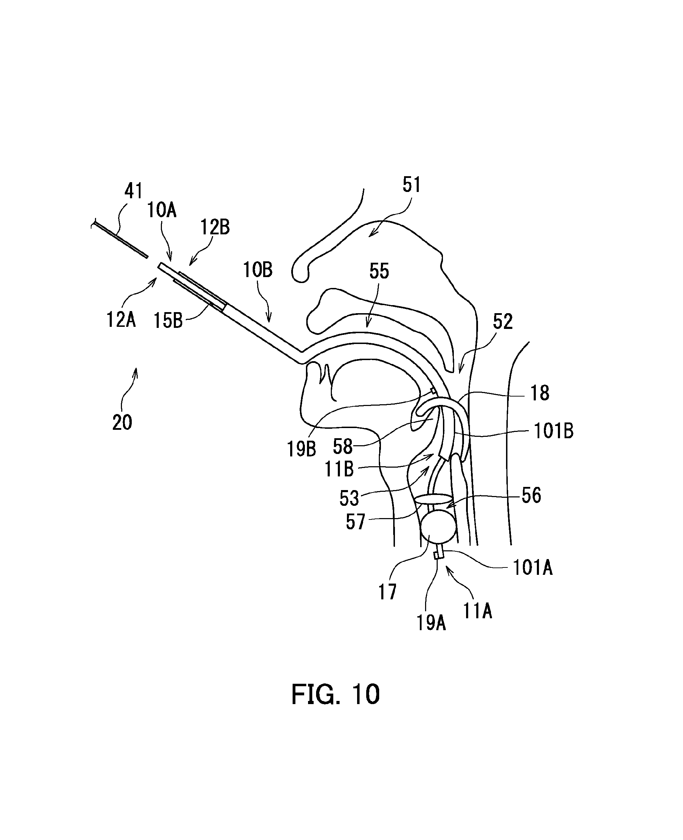

The guide device 20 includes a single guide tube 10 in the embodiment described with reference to FIGS. 8 and 9A-9D, which however, should not be taken to limit the present invention, as will be described with reference to FIG. 10. The guide device 20 may include one or more guide tubes 10.

### [Second Embodiment of Guide Device]

FIG. 10 is a schematic illustration showing the second embodiment of the guide device 20 according to the present invention. The guide device 20 in the present embodiment includes an inner guide tube 10A, an outer guide tube 10B, and a form changing means (not shown). Note that the oral cavity 55, the pharyngeal cavity 52, the laryngeal cavity 53, and a trachea 56 collectively correspond to the internal space.

The outer guide tube 10B forms a path used for inserting the inner guide tube 10A into the internal space. The inner guide tube 10A forms a path for delivery of a to-be-delivered object that performs a predetermined operation on the trachea 56. In the present embodiment, the outer guide tube 10B and the form changing means 70 are the same as those of the guide device in the embodiment described with reference to FIGS. 8, 9A-9D, and therefore, detailed description of them is omitted. Further, by the method described with reference to FIGS. 9A-9D, a first end portion 11B of the outer guide tube 10B can be inserted up to the laryngeal cavity 53.

The inner guide tube 10A includes a first end portion 11A, a second end portion 12A, and a tube inner space 15A. The first end portion 11A of the guide tube 10 is inserted in a tube internal space 15B of the outer guide tube 10B to be inserted in the trachea 56 through the outer guide tube 10B. The inner guide tube 10A has a predetermined initial form that allows the first end portion 11A, which extends out from the first end portion 11B of the outer guide tube 10B, to be inserted to the trachea 56 via vocal cords 57. Specifically, the initial form of the inner guide tube 10A is designed so as to conform with the curved shape of the space from the first end portion 11B of the outer guide tube 10B to a target point in the trachea 56.

For example, in observation of the trachea 56 as a predetermined operation, the tip end of the insertion portion 41 of the endoscope is inserted from a second end portion 12B of the inner guide tube 10A to be allowed to pass through the oral cavity 55, the pharyngeal cavity 52, and the laryngeal cavity 53. Then, the insertion portion 41 of the endoscope is projected out from the first end portion 11B of the inner guide tube 10A for observation of the trachea 56.

The method for using the guide device 20 has been described with reference to FIG. 10. The guide device 20 in the present embodiment includes the inner and outer guide tubes 10A and 10B having the respective predetermined initial forms. A combination of a plurality of guide tubes having different initial forms can form a delivery path for the predetermined operation even on a body cavity of which space complicatedly winds.

Note that the inner guide tube 10A of the guide device 20 according to the present embodiment may additionally include a first cuff 17, or the outer guide tube 10B may additionally include a second cuff 18.

The first cuff 17, which is expandable and shrinkable, is provided around a main body 101A of the inner guide tube 10A. When the first cuff 17 is expanded when in use of the guide device 20, the trachea can be obturated.

The second cuff 28, which is expandable and shrinkable, is provided around a main body 101B of the outer guide tube 10B. The second cuff 18 is in a parachute form when expanded. When the second cuff 18 is expanded when in use of the guide device 20, the vicinity of an epiglottis 58 in the laryngeal cavity 53 can be obturated to separate the respiratory tract continuing from the oral cavity and nasal cavity from the esophagus.

Any known configuration is employable as each configuration of the first and second cuff 17 and 18. Therefore, no detailed description and illustration are given. For example, a fluid transporting path (not shown) communicating with the first cuff 17 (or the second cuff 18) is formed in the inner guide tube 10A (or the outer guide tube 10B). When a fluid (e.g., air or water) is delivered to or from the first or second cuff 17 or 18 through the fluid transporting path, the first or second cuff 17 or 18 can be expanded or shrunk.

As has been discussed with reference to FIG. 10, the guide device 20 according to the present embodiment additionally includes the first and second cuffs 17 and 18. When in use of the guide device 20, expansion of the first cuff 17 can fix the inner guide tube 10A to the trachea 56 and reduce leakage of air in the trachea 56 (e.g., an anesthetic gas sent through the inner guide tube 10A). Further, in the present embodiment, the expanded second cuff 18 when in use of the guide device 20 can prevent saliva and/or flowing-back gastric juice from flowing into a lung through the trachea 56 and from being retained between the vocal cords 57 and the first cuff 17. Thus, an outbreak of pneumonia can be prevented that may be caused due to the use of the guide device 20.

Further, the guide device 20 of the present embodiment may additionally include an imaging device 19A provided at the inner guide tube 10A and/or an imaging device 19B provided at the outer guide tube 10B. The imaging device 19A is arranged on the outer surface of the main body 101A of the inner guide tube 10A. For example, the imaging device 19A is arranged on the side of the first cuff 17 where the first end portion 11A of the inner guide tube 10A is located. The imaging device 19B is arranged on the outer surface of the main body 101B of the outer guide tube 10B. For example, the imaging device 19B is arranged on the side of the second cuff 18 where the second end portion 12B of the outer guide tube 10B is located. Cables to connect the imaging device 19A (or the imaging device 19B)) to an external device (e.g., a device for displaying or processing images) are arranged in the tube inner space of the inner guide tube 10A (or the tube inner space of the outer guide tube 10B). Alternatively, like the main body 101 of the guide tube 10 in which the insertion portion 41 of the endoscope is integrally embedded in the embodiment described with reference to FIG. 5, the imaging device 19A (or the imaging device 19B) may be integrally embedded in the main body 101A of the inner guide tube 10A (or the main body 101B of the outer guide tube 10B) also in the present embodiment.

As described with reference to FIG. 10, the guide device 20 in the present embodiment may additionally include the imaging device 19A and/or the imaging device 19B. Thus, an operator can insert the guide tube into a body cavity, while observing the body cavity. In addition, the form changing means 70 can function as a tongue depressor for depressing the root of a tongue, thereby further facilitating insertion of the guide tube 10. Accordingly, the guide tube and the first and second cuffs 17 and 18 can be accurately set at respective desired locations without using a laryngoscope. This can reduce damage to the teeth caused due to the use of a laryngoscope, an edema of the larynx including the vocal cords, and an inflammation of the vocal cords.

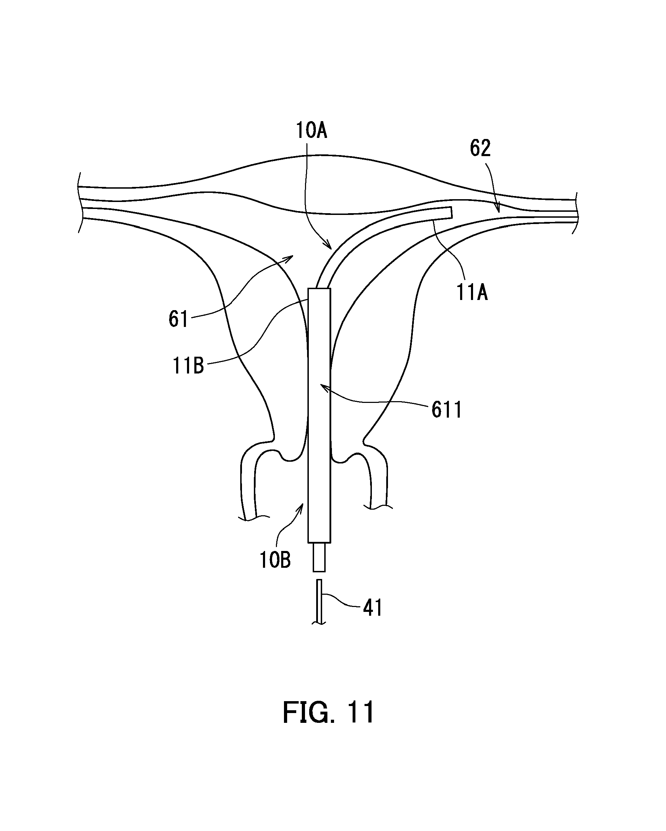

Note that the guide device 20 in the embodiment described with reference to FIG. 10 is employed in the case where the oral cavity 55, the pharyngeal cavity 52, the laryngeal cavity 53, and the trachea 56 collectively correspond to the internal space, which however, should not be taken to limit the present invention, as described with reference to FIG. 11. FIG. 11 is a schematic illustration showing the guide device 20 employed in observation of an oviduct 62 as a predetermined operation. In observation of the oviduct 62, the outer guide tube 10B forms a path to allow the inner guide tube 10A to be inserted into a uterine cavity 61. The inner guide tube 10A forms a path to allow an insertion portion 41 of an endoscope to be inserted into the vicinity of the inlet of the oviduct 62.

The outer guide tube 10B has a predetermined initial form that allows the outer guide tube 10B to be inserted into the uterine cavity 61 through a vagina (not shown) and a portio vaginalis uteri 611. Specifically, the initial form of the outer guide tube 10B is designed so as to conform with a winding shape of the vagina and the portio vaginalis uteri 611. By contrast, the inner guide tube 10A has a predetermined initial form that allows the first end portion 11A extending from the first end portion 11B of the outer guide tube 10B to be inserted up to the vicinity of the inlet of the oviduct 62. Specifically, the initial form of the inner guide tube 10A is designed so as to conform with a winding shape of the space from the first end portion 11B of the outer guide tube 10B to the vicinity of the inlet of the oviduct 62. As shown in FIG. 11, the guide device 20 in the present embodiment is employable in the case where the uterine cavity 61 corresponds to the internal space. Though not shown, even the guide device 20 employed in observation of the oviduct 62 can include the imaging device(s) at the inner guide tube 10A and/or the outer guide tube 10B.

### [INDUSTRIAL APPLICABILITY]

The guide tube and the guide device according to the present invention can be used suitably for forming a delivery path for a to-be-delivered object. The to-be-delivered object is an object used for a predetermined operation on a portion of interest in an internal space. For example, a path through which an endoscope, air, or a therapeutic agent can be delivered to a body cavity can be formed in the medical fields. Alternatively, a path through which an endoscope is delivered to an internal space of a facility or piping can be formed in the industrial fields.

### [REFERENCE SIGNS LIST]

- 10: Guide tube
- 101: Main body
- 11: First end portion
- 12: Second end portion
- 13: Curved portion
- 131: First curved portion
- 132: Second curved portion
- 14: Straight portion
- 141: Axial line
- 15: Tube inner space
- 16: Closing member
- 18: First cuff
- 19: Second cuff
- 20: Guide device
- 30: Internal space
- 31: One end portion
- 32: Other end portion
- 33: Winding portion
- 41: Insertion portion
- 42: Syringe
- 421: Needle
- 51: Nasal cavity
- 52: Pharyngeal cavity
- 53: Laryngeal cavity
- 54: Nostril
- 55: Oral cavity
- 56: Trachea
- 57: Vocal cords
- 58: Epiglottis
- 61: Uterine cavity
- 62: Oviduct
- 70: Form changing means

## Claims

1. A guide tube having an initial form designed in advance according to a shape of an internal space, wherein
when an external force is applied to the guide tube, the guide tube deforms from the initial form to another form, and
when the applied external force is removed, the guide tube returns to the initial form.

2. The guide tube of claim 1, comprising:
at least one curved portion.

3. The guide tube of claim 2, further comprising:
a straight portion, a first curved portion, and a second curved portion,
wherein the straight portion continues to the first curved portion, and the first curved portion continues to the second curved portion,
the first curved portion extends away from an axial line of the straight portion from an end of the straight portion, and
the second curved portion extends to approach the axial line of the straight portion from an end of the first curved portion.

4. The guide tube of any one of claims 1-3, comprising:
a first end portion,
wherein the first end portion is closed.

5. The guide tube of claim 4, wherein
a closing member configured to close the first end portion is provided at the first end portion.

6. The guide tube of claim 5, wherein
the closing member is detachable.

7. The guide tube of any one of claims 1-6, comprising:
an inner guide tube and an outer guide tube.

8. The guide tube of claim 7, wherein
an imaging device is provided at one or each of the inner and outer guide tubes.

9. A guide device comprising:
the guide tube of any one of claims 1-6; and
a form changing means configured to apply an external force to the guide tube.

10. The guide device of claim 9, wherein
the guide tube includes an inner guide tube and an outer guide tube, and
the inner guide tube is inserted in the outer guide tube.

11. The guide device of claim 10, wherein
the inner guide tube includes a first cuff, and the outer guide tube includes a second cuff.

12. The guide device of claim 10 or 11, further comprising:
an imaging device provided at one or each of the inner and outer guide tubes.

13. The guide device of any one of claims 9-12, wherein
the form changing means is a stem.

14. The guide device of any one of claims 9-12, wherein
the form changing means is a fluid.

15. A method for using the guide device of any one of claims 9-14, comprising:
deforming the guide tube from the initial form to the other form and maintaining the guide tube in the other form by applying an external force to the guide tube using the form changing means;
inserting the guide tube into the internal space;
returning the guide tube to the initial form by removing the external force applied to the guide tube; and
adjusting a position of the guide tube.

16. The method of claim 15, wherein
the returning guide tube to the initial form and the adjusting a position of the guide tube are performed in plural stages.
